# EUROPEAN PATENT APPLICATION

(11) **EP 4 286 446 A1**
(43) Date of publication of application: **06.12.2023**
(21) Application number: 22460070.0
(22) Date of filing: 15.12.2022
(51) Int. Cl.: C08G 61/12, C08L 65/00, C09D 165/00, H10K 85/10, H10K 85/60

(54) **A NOVEL PERINONE POLYMER AND A METHOD OF OBTAINING A PERINONE POLYMER IN THE PROCESS OF POTENTIOSTATIC POLYMERIZATION**

(30) Priority: 03.06.2022 PL 44137222
(71) Applicant: Politechnika Slaska, 44-100 Gliwice (PL)
(72) Inventor: Lapkowski, Mieczyslaw, 44-207 Rybnik (PL); Janasik, Patryk, 56 Orzesze (PL); Czichy, Malgorzata, 40-404 Katowice (PL)

(57) **Abstract**

A new perinone polymer with the general formula containing units marked as s, t, u, v, w, x, y, z: 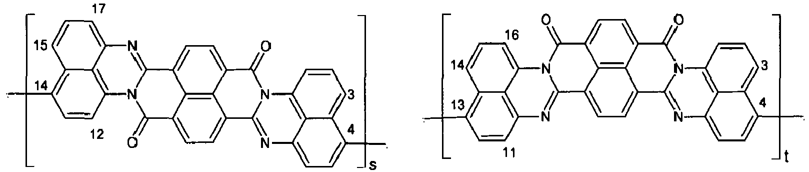 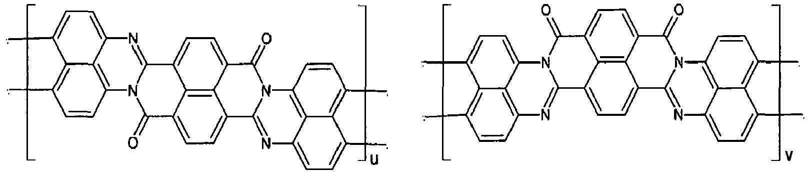 in anodic mode 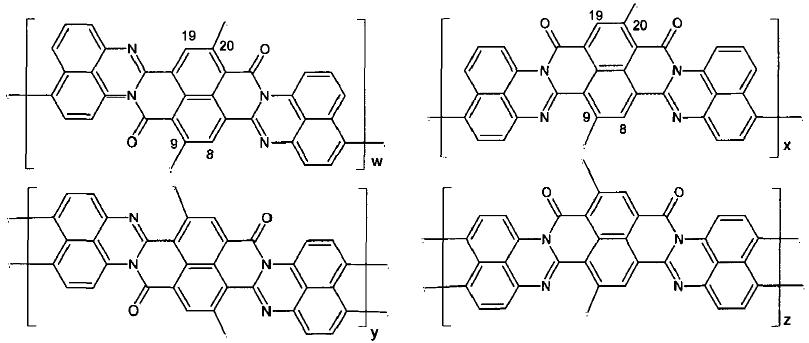 in anodic-cathodic mode
where:
s - poly(*anti*M) by single bond via positions 1, 2, 3, 4, 5, 6, 12, 13, 14, 15, 16 and/or 17
t - poly(*syn*M) by single bond via positions 1, 2, 3, 4, 5, 6, 11,12, 13, 14, 15 and/or 16
u - poly(*anti*M) by double bond via positions 1, 2, 3, 4, 5, 6, 12, 13, 14, 15, 16 and/or 17
v - poly(*syn*M) by double bond via positions 1, 2, 3, 4, 5, 6, 11,12, 13, 14, 15 and/or 16
w - poly(*anti*M) by single bond via positions 1, 2, 3, 4, 5, 6, 8, 9, 12, 13, 14, 15, 16, 17, 19 and/or 20
x- poly(*syn*M) by single bond via positions 1, 2, 3, 4, 5, 6, 8, 9, 11,12, 13, 14, 15, 16, 19 and/or 20
y - poly(*anti*M) by double bond via positions 1, 2, 3, 4, 5, 6, 8, 9, 12, 13, 14, 15, 16, 17, 19 and/or 20
z - poly(*syn*M) by double bond via positions 1, 2, 3, 4, 5, 6, 8, 9, 11, 12, 13, 14, 15, 16, 19 and/or 20.

The invention also relates to a method of obtaining a perinone polymer in the process of potentiostatic polymerization.

## Description

The subject of the patent is a novel perinone polymer and a method of obtaining a perinone polymer in the process of potentiostatic polymerization.

Π-conjugated polymers have wide range of applications due to their ability of electrical conduction. This property can be tailored by physical agents and / or chemical modifications (Dengke Zhaoa, Ligui Li, Wenhan Niua, Shaowei Chena, Highly conductive polythiophene films doped with chloroauric acid for dual-mode sensing of volatile organic amines and thiols, Sensors and Actuators B: Chemical 2017, 243, 380-387, doi: 10.1016/j.snb.2016.12.018). The increase in electrical conductivity could be of a mixed nature in which electron holes, electrons, ions or electron-hole pairs (so-called excitons) serve as the current carriers. In the case of semiconductors, controlling the conductive properties is crucial because it enables their specific application in electronics and optoelectronics. Doping process increases both the electronic and ionic conductivity of organic semiconductors, but only a few of polymers can be reduced, and even fewer of them demonstrate stable conductivity in the reduced state. Therefore, stable electron conductors that are effective in a neutral state and functional at room temperature are particularly challenging to obtain. The fundamental problem is to achieve a π-conjugated polymer with high electron conductivity in the neutral state. Increasing the electron conductivity of π-conjugated polymers can be attain by increasing the effective delocalization length of the main chain, side substituent effects, increasing long-range interactions, obtaining a donor-acceptor, ladder or partially ladder structure e.g. poly(benzobisimidazobenzophenanthroline) BBL (CLP - Conjugated Ladder Polymers). CLPs differ from conventional conductive polymers in that their backbones contain fused aromatic rings. Torsional rotation between aromatic units along the conduted backbone is restricted by this structure, for example, ladder polymers based on polyquinoxaline (J. K. Stille and E. L. Mainen , J. Polym. Sci., Part B: Polym. Lett., 1966, 4, 39 -41), and poly(phenothiazine) (M. D. Pace, O. K. Kim, (1988). Conducting properties and electron paramagnetic resonance of polyphenothiazine and polyphenoxazine ladder polymers, Synth. Met, 1988, 25, 333-339. doi:10.1016/0379-6779(88)90573-5), poly(phenothiazine), and carbazole. Findings in the literature also reports the reactivity of perinone monomers obtained by condensation from 1,8-naphthalenediamine (Czichy, M.; Motyka, R.; Zassowski, P.; Grabiec, E.; Janasik, P.; Brzeczek-Szafran, A.; Laba, K.; Wolinska-Grabczyk, A.; Lapkowski, M. Effects of solution-phase ordering on the spectroscopic properties and electrooxidative reactivity of isomeric mixtures and isolated isomers of synthesized amidine derivatives. Dye Pigment. 2020, 178, 108309 and Czichy, M.; Janasik, P.; Motyka, R.; Zassowski, P.; Grabiec, E.; Wolinska-Grabczyk, A.; Lapkowski, M. Influence of isomeric phthaloperinone monomers on the formation of π-dimers and σ-bonded segments in electrochemically-crosslinked products. Electrochim. Acta 2021, 370, 137669). For this reason, we have proposed novel perinons with perimidine terminal groups as polymer precursors, since this one consists of both a π-donor and a π-acceptor system where, lone pairs of nitrogen atoms transfer their electron density to the naphthalene ring. Therefore, there increases the simultaneously occurrence of electrophilic and nucleophilic properties and subsequent oxidative coupling reactions. The invention relates to a perinone polymer characterized by electroactivity, bipolarity, n-conductivity and a low band gap. This polymer can be used in the production of: diodes (OLED, NIR), (bio)sensors, memory devices, substitutes for metallic conductors, anti-corrosion coatings, etc. ( R. L. Van Deusen, J. Polym. Sci., Part B: Polym. Lett., 1966, 4, 211-214).

The aim of the invention is to develop a perinone polymer and a method of its preparation, which will allow to obtain a material with a high n-type conductivity and a low band gap.

The content of this invention is a novel perinone polymer, and its general formula contains units marked as u, w, v, x, y, z: 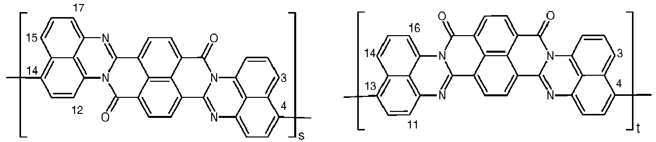 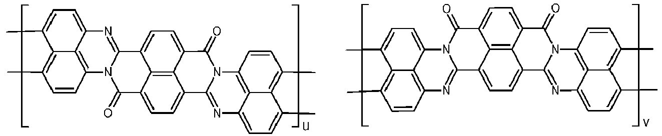 in anodic mode 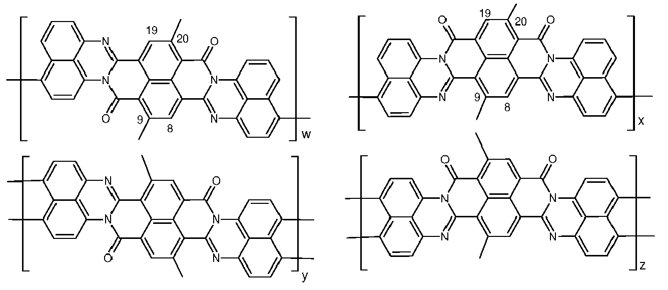 in anodic-cathodic mode
where:
s - poly(*anti*M) by single bond via positions 1, 2, 3, 4, 5, 6, 12, 13, 14, 15, 16 and/or 17
t - poly(*syn*M) by single bond via positions 1, 2, 3, 4, 5, 6, 11,12, 13, 14, 15 and/or 16
u - poly(*anti*M) by double bond via positions 1, 2, 3, 4, 5, 6, 12, 13, 14, 15, 16 and/or 17
v - poly(*syn*M) by double bond via positions 1, 2, 3, 4, 5, 6, 11,12, 13, 14, 15 and/or 16
w - poly(*anti*M) by single bond via positions 1, 2, 3, 4, 5, 6, 8, 9, 12, 13, 14, 15, 16, 17, 19 and/or 20
x- poly(*syn*M) by single bond via positions 1, 2, 3, 4, 5, 6, 8, 9, 11,12, 13, 14, 15, 16, 19 and/or 20 y - poly(*anti*M) by double bond via positions 1, 2, 3, 4, 5, 6, 8, 9, 12, 13, 14, 15, 16, 17, 19 and/or 20
z - poly(*syn*M) by double bond via positions 1, 2, 3, 4, 5, 6, 8, 9, 11, 12, 13, 14, 15, 16, 19 and/or 20 z - poli(*syn*M) poprzez dwa wi 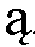 zanie za pośrednictwem pozycji 1, 2, 3, 4, 5, 6, 8, 9, 11, 12, 13, 14, 15, 16, 19 i/lub 20.

The content of the invention is also a method of obtaining a perinone polymer in the process of potentiostatic polymerization in the mixture of *anti*M+*syn*M (*anti* benzo [*lmn*] diperimidino [2,1-*b*:2',1'-*i*] [3,8] phenanthroline- 10,21-dione and *syn* benzo[*lmn*]diperimidino[2,1-*b*:1',2'-*j*][3,8]phenanthroline-18,21-dione in a molar ratio from 1:0 to 0: 1), using chronoamperometry on a working electrode made of metallic or carbon materials; preferably platinum, a platinum alloy or a glassy carbon, where the process is carried out under the conditions of:
- polarization including potential values within the oxidation peak of *anti*M+*syn*M (anodic mode) in the range of -0.5 to 1 V against the redox ferrocene/ferrocenium redox pair,
- after the anodic mode the product is polarized with potential in the range between -1.75 and 0.5 V (cathode) against the redox ferrocene/ferrocenium redox pair (anodic-cathodic mode),
- deposition from the solution *anti*M+*syn*M with solvent as toluene or benzene and electrolyte as tetrabutylammonium tetrafluoroborate, tetrabutylammonium hexafluorophosphate or tetraammonium hexafluorophosphate,
- deposition from solid state of *anti*M+*syn*M in solvent as dichloromethane, chlorobenzene, 1,2-dichlorobenzene or methanol with electrolyte as form of tetrabutylammonium tetrafluoroborate, tetrabutylammonium hexafluorophosphate, tetraethylammonium hexafluorophosphate,
the process is carried out until the PPBF product is deposited on the working electrode.

### Example I.

The perinone polymer with a poly(perimidinobenzophenanthroline) skeleton (PPBF) was obtained by electropolymerization from a mixture of benzo[*lmn*]diperimidino[2,1-*b*:2',1'-*i*][3,8]phenanthroline-10,21-dione and benzo[*lmn*]diperimidino[2,1-*b*:1',2'-*j*][3,8]phenanthroline-18,21-dione. The PPBF polymer is electrically conductive, has complex redox activity, mixed conductivity and a low bad gap.

### Materials

The following materials were used: naphthalene-1,4,5,8-tetracarboxylic dianhydride (1), 1,8-naphthalenediamine (2); tetrabutylammonium tetrafluoroborate; dichloromethane; toluene; benzene; chlorobenzene; 1,2-dichlorobenzene; nitrobenzene; methanol and isobutanol.

### Synthesis of monomers antiM+synM

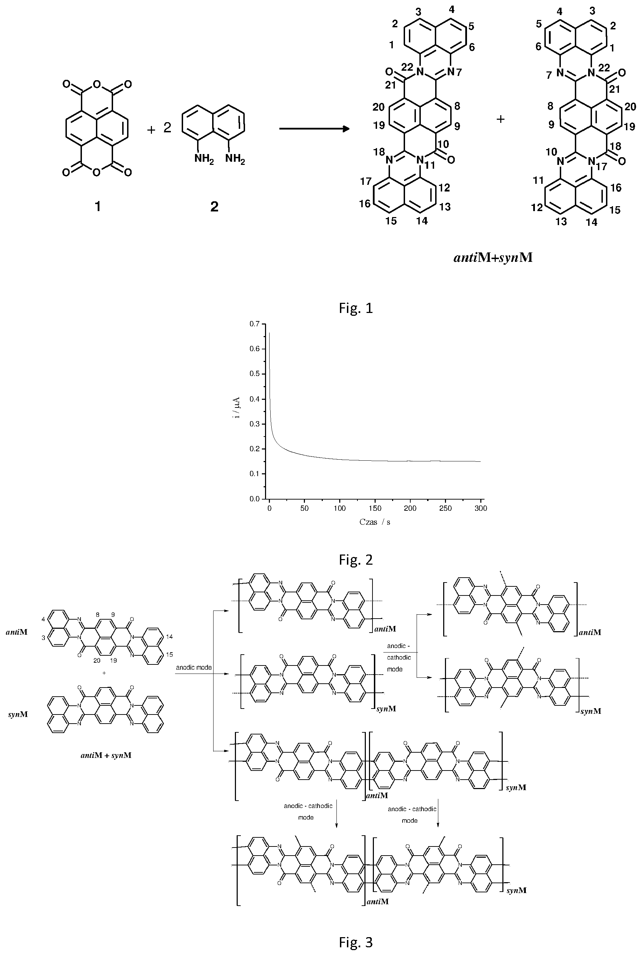

Mixture of monomers (*anti*M+synM) as a mixture of *anti* isomer benzo[*lmn*]diperimidino[2,1-*b*:2',1'-*i*][3,8]phenanthroline-10,21-dione and *syn* isomer benzo[*lmn*]diperimidino[2,1-*b*:1',2'-*j*][3,8]phenanthroline-18,21-dione was obtained by condensation reaction 1 compound (0.270 g, 1 mmol) with 2 compound (0.316 g, 2 mmols) (FIG. 1). The reaction was carried out in 10 ml of isobutanol for 24h under reflux. After the reaction was complete, the mixture was allowed to cool to room temperature. The precipitate was then filtered off and washed with methanol and then was first heated up in hexane for 1h and after in methanol for 1h under reflux. The precipitate was filtered off and dried under vacuum. *Anti*M+*syn*M was obtained as a black powder (0,399 g, 78%) Tₘ: >450 °C. ¹H-NMR: δ/ppm (300 MHz, CDCl₃ with the addition of 10%-CF₃COOD, Me₄Si, mixture of *anti* and *syn* isomers) = 1.05 (CF₃COOH); 9.28 (m, 2H); 9.05 (m, 2H); 8.59 (m, 2H); 7.76 (m, 4H); 7.65 (m, 2H); 7.56-7.48 (m, 4H). IR: νₘₐₓ/cm⁻¹ = 1678, 1624, 1595, 1548, 1528, 1492, 1469, 1394, 1369, 1342, 1319, 1284, 1257, 1225, 1165, 1121, 1106, 1072, 1043, 1003, 905, 865, 829, 793, 752.

FIG.1. Diagram showing the synthesis of *anti*M+*syn*M monomer mixture for the preparation of the PPBF polymer.

### Preparation of PPBF polymer

PPBF is obtained under potentiostatic polymerization (chronoamperometry) in a solution of *anti*M+*syn*M and an electrolyte, preferably tetrabutylammonium tetrafluoroborate in dichloromethane, chlorobenzene or 1,2-dichlorobenzene. PPBF polymer can be deposited on a variety of metallic, carbon, organic and inorganic electrically conductive surfaces. Obtaining of PPBF by chronoamperometric method is carried out using a constant polarization, where working electrode is polarized with a potential above the start of the first oxidation peak of *anti*M+*syn*M, until the product precipitates on the electrode (FIG. 2). The polymerization can also be carried out in the solid state using the potentiodynamic or potentiostatic method.

PPBF polymer is characterized by wide charging currents in the potential range from -2 V to 1.7 V with reversible redox pairs at 0.10/0.15 V; 0.35/0.25 V, -0.95/-1.00 and -1.35/-1.40 V against the redox ferrocene/ferrocenium pair. The levels of the HOMO, LUMO and LUMO+1 orbitals were calculated for the *anti*M+*syn*M precursors and alternative products, and thus the poly(perimidinobenzophenanthroline) skeleton structure was found in the PPBF product as shown in FIG. 3.

FIG. 2. Potentiostatic method: chronoamperogram of the PPBF polymerization process.

FIG. 3 Diagram of the mechanism of electrochemical preparation of PPBF and possible structure of PPBF obtained in anode and anodic-cathodic modes.

### Method for PPBF obtaining

A mixture of *anti*M+*syn*M monomers *(anti* benzo[*lmn*]diperimidino[2,1-b:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio in the range of 1:0 to 0:1), is subjected to potentiostatic polymerization (chronoamperometry) under the following conditions:
- polarization with a constant potential including potential values within the oxidation peak of *anti*M+*syn*M (anodic mode) in the range of -0.5 to 1 V against the redox ferrocene/ferrocene pair,
- after the anodic mode, the product is polarized in the potential range from -1.75 to 0.5 V (cathodic) against the redox ferrocene/ferrocene pair (anodic-cathodic mode),
- material of the working electrode - metallic or carbon materials, e.g. platinum, platinum alloys, glassy carbon etc.,
- deposition from solution of *anti*M+*syn*M, preferably toluene, benzene, and an electrolyte that is dissolved in a solvent, preferably tetrabutylammonium tetrafluoroborate, tetrabutylammonium hexafluorophosphate or tetraethylammonium hexafluorophosphate,
- deposition from solid state of *anti*M+*syn*M, solvents, e.g. dichloromethane, chlorobenzene, 1,2-dichlorobenzene, methanol and electrolyte which is dissolved in a solvent, e.g. tetrabutylammonium tetrafluoroborate, tetrabutylammonium hexafluorophosphate, tetraethylammonium hexafluorophosphate,
- time of the process - up to the precipitation of the PPBF product on the working electrode.

As a result of the method, depending on the mode type (anodic or anodic-cathodic mode), the following perinone polymer was formed with the formula: 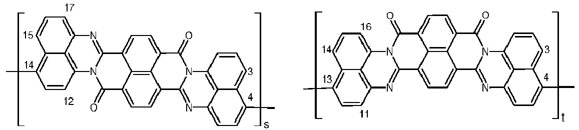 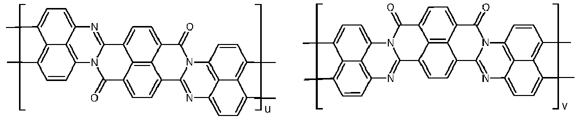 in anodic mode 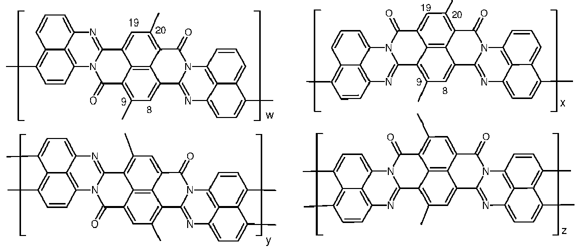 in anodic-cathodic mode
where:
s - poly(*anti*M) by single bond via positions 1, 2, 3, 4, 5, 6, 12, 13, 14, 15, 16 and/or 17
t - poly(*syn*M) by single bond via positions 1, 2, 3, 4, 5, 6, 11,12, 13, 14, 15 and/or 16
u - poly(*anti*M) by double bond via positions 1, 2, 3, 4, 5, 6, 12, 13, 14, 15, 16 and/or 17
v - poly(*syn*M) by double bond via positions 1, 2, 3, 4, 5, 6, 11,12, 13, 14, 15 and/or 16
w - poly(*anti*M) by single bond via positions 1, 2, 3, 4, 5, 6, 8, 9, 12, 13, 14, 15, 16, 17, 19 and/or 20
x- poly(*syn*M) by single bond via positions 1, 2, 3, 4, 5, 6, 8, 9, 11,12, 13, 14, 15, 16, 19 and/or 20
y - poly(*anti*M) by double bond via positions 1, 2, 3, 4, 5, 6, 8, 9, 12, 13, 14, 15, 16, 17, 19 and/or 20
z - poly(*syn*M) by double bond via positions 1, 2, 3, 4, 5, 6, 8, 9, 11, 12, 13, 14, 15, 16, 19 and/or 20

### Example II.

Example II differs from the I in that the PPBF is obtained using the following procedure.

A mixture of *anti*M+*syn*M monomers (*anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio of 1:0), is subjected to ppotentiostatic polymerization (chronoamperometry) under the conditions:
- polarization with a constant potential including potential values within the oxidation peak of *anti*M+*syn*M (anodic mode) at -0.5 V against the redox ferrocene/ferrocene pair,
- after the anodic mode, the product is polarized at -1.75 V (cathodic) against the redox ferrocene/ferrocene pair (anodic-cathodic mode),
- material of the working electrode - platinum,
- deposition from the solution of *anti*M+*syn*M in toluene with tetrabutylammonium tetrafluoroborate electrolyte, which is dissolved in this solvent,
- deposition from solid state of *anti*M+*syn*M, in dichloromethane with tetrabutylammonium tetrafluoroborate electrolyte, which is dissolved in this solvent,
- time of the process - up to the precipitation of the PPBF product on the working electrode.

### Example III.

Example III differs from the I in that PPBF is obtained using following procedure.

A mixture of *anti*M+*syn*M monomers (*anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio of 0:1), is subjected to potentiostatic polymerization (chronoamperometry) under the conditions:
- polarization with a constant potential including potential values within the oxidation peak of *anti*M+*syn*M (anodic mode) at 1 V against the redox ferrocene/ferrocene pair,
- after the anodic mode, the product is polarized at 0.5 V (cathodic) against the redox ferrocene/ferrocene pair (anodic-cathodic mode),
- material of the working electrode - platinum alloy,
- deposition from the solution of *anti*M+*syn*M in benzene with tetrabutylammonium hexafluorophosphate electrolyte, which is dissolved in this solvent,
- deposition from solid state of *anti*M+*syn*M, in chlorobenzene with tetrabutylammonium hexafluorophosphate electrolyte, which is dissolved in this solvent,
- time of the process - up to the precipitation of the PPBF product on the working electrode.

### Example IV.

Example IV differs from the I in that the PPBF is obtained using following procedure.

A mixture of *anti*M+*syn*M monomers *(anti* benzo[*lmn*]diperimidino[2,1-*b*:2',1'-*i*][3,8]phenanthroline-10,21-dione and *syn* benzo[*lmn*]diperimidino[2,1-*b*:1',2'-*j*][3,8]phenanthroline-18,21-dione, preferably in a molar ratio of 0.5:0.5), is subjected to ppotentiostatic polymerization (chronoamperometry) under the conditions:
- polarization with a constant potential including potential values within the oxidation peak of *anti*M+*syn*M (anodic mode) at 0.2 V against the redox ferrocene/ferrocene pair,
- after the anodic mode, the product is polarized at -0.3 V (cathodic) against the redox ferrocene/ferrocene pair (anodic-cathodic mode),
- material of the working electrode - glassy carbon,
- deposition from the solution of *anti*M+*syn*M in benzene with tetraethylammonium hexafluorophosphate electrolyte, which is dissolved in this solvent,
- deposition from solid state of *anti*M+*syn*M, in methanol with tetraethylammonium hexafluorophosphate electrolyte, which is dissolved in this solvent,
- time of the process - up to the precipitation of the PPPI product on the working electrode.

## Claims

1. A new perinone polymer with the general formula containing units marked as s, t, u, v, w, x, y, z: 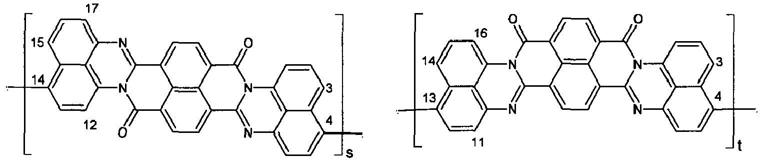 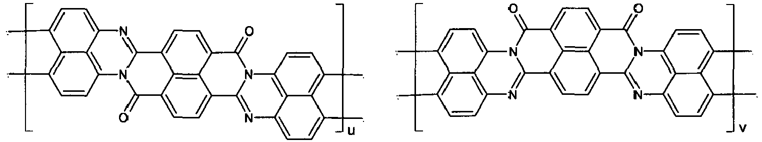 in anodic mode 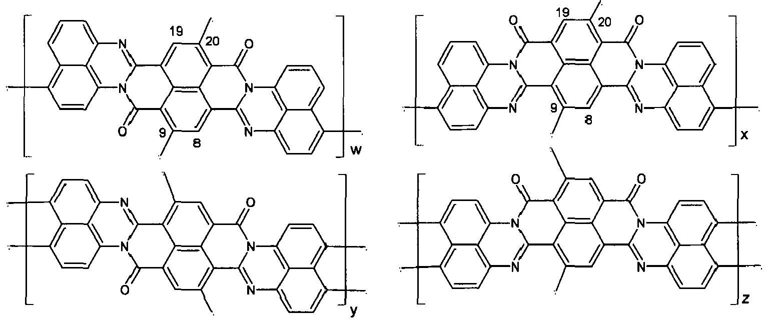 in anodic-cathodic mode
where:
s - poly(*anti*M) by single bond via positions 1, 2, 3, 4, 5, 6, 12, 13, 14, 15, 16 and/or 17
t - poly(*syn*M) by single bond via positions 1, 2, 3, 4, 5, 6, 11,12, 13, 14, 15 and/or 16
u - poly(*anti*M) by double bond via positions 1, 2, 3, 4, 5, 6, 12, 13, 14, 15, 16 and/or 17
v - poly(*syn*M) by double bond via positions 1, 2, 3, 4, 5, 6, 11,12, 13, 14, 15 and/or 16
w - poly(*anti*M) by single bond via positions 1, 2, 3, 4, 5, 6, 8, 9, 12, 13, 14, 15, 16, 17, 19 and/or 20
x- poly(*syn*M) by single bond via positions 1, 2, 3, 4, 5, 6, 8, 9, 11,12, 13, 14, 15, 16, 19 and/or 20
y - poly(*anti*M) by double bond via positions 1, 2, 3, 4, 5, 6, 8, 9, 12, 13, 14, 15, 16, 17, 19 and/or 20
z - poly(*syn*M) by double bond via positions 1, 2, 3, 4, 5, 6, 8, 9, 11, 12, 13, 14, 15, 16, 19 and/or 20.

2. Method for obtaining a perinone polymer by potentiodynamic polymerization, wchich is characterized as the mixture of *anti*M+*syn*M monomers (*anti* benzo*[lmn*]diperimidino[2,1-*b*:2',1'-*i*][3,8]phenanthroline-10,21-dione and *syn* benzo*[lmn*]diperimidino[2,1-*b*:1',2'-*j*][3,8]phenanthroline-18,21-dione, preferably in a molar ratio in the range 1:0 to 0:1), using potentiostatic polymerization (chronoamperometry) on a working electrode made of metallic or carbon materials; preferably platinum, a platinum alloy or a glassy carbon, where the process is carried out under the following conditions:
- polarization with a constant potential including potential values within the oxidation peak of *anti*M+*syn*M (anodic mode) in the range of -0.5 to 1 V against the redox ferrocene/ferrocene pair,
- after the anodic mode, the product is polarized in the potential range from - 1.75 to 0.5 V (cathodic) against the redox ferrocene/ferrocene pair (anodic-cathodic mode),
- deposition from solution of *anti*M+*syn*M, preferably toluene, benzene, and an electrolyte that is dissolved in a solvent, preferably tetrabutylammonium tetrafluoroborate, tetrabutylammonium hexafluorophosphate or tetraethylammonium hexafluorophosphate,
- deposition from solid state of *anti*M+*syn*M, solvents, e.g. dichloromethane, chlorobenzene, 1,2-dichlorobenzene, methanol and electrolyte which is dissolved in a solvent, e.g. tetrabutylammonium tetrafluoroborate, tetrabutylammonium hexafluorophosphate, tetraethylammonium hexafluorophosphate,
the process is carried out until the PPBF product is precipitated on the working electrode.
